# FASCICULE DE BREVET EUROPEEN

(11) **EP 0 720 424 B1**
(45) Date de publication et mention de la délivrance du brevet: **07.01.1999**
(21) Numéro de dépôt: 94928424.4
(22) Date de dépôt: 21.09.1994
(51) Int. Cl.: A01H 4/00, C12N 5/00

(54) **PROCEDE POUR FAVORISER LA DIFFERENCIATION DE CELLULES VEGETALES**
VERFAHREN ZUR FÖRDERUNG DER PFLANZENDIFFERENZIERUNG
METHOD FOR ENCOURAGING PLANT CELL DIFFERENTIATION

(30) Priorité: 22.09.1993 FR 9311288
(43) Date de publication de la demande: 10.07.1996
(73) Titulaire: L.V.M.H. RECHERCHE, F-92000 Nanterre (FR)
(72) Inventeur: MAES, Olivier, Saskatoon, Saskatchewan S7N 2S2 (CA); JOUENNE, Thierry, F-76000 Rouen (FR); GUERN, Jean, F-91190 Gif-sur-Yvette (FR); COUTOS-THEVENOT, Pierre, F-75005 Paris (FR)
(74) Mandataire: Ahner, Francis
(86) Numéro de dépôt international: FR9401104
(87) Numéro de publication internationale: WO9508261

(56) Documents cités:
- EP-A- 0 293 598
- EP-A- 0 455 597
- FR-A- 2 537 157
- US-A- 4 818 693
- J. PLANT PHYSIOL., vol.130, no.1, 1987 pages 49 - 55 R.N. TRIGIANO ET AL. 'Regulation of Growth and Somatic Embryogenesis by Proline and Serine in Suspension Cultures of Dactylis glomerata'
- PLANT CELL REP., vol.3, no.5, 1984 pages 210 - 214 V.N. RONCHI ET AL. 'Stimulation of carrot somatic embryogenesis by proline and serine'
- PLANT CELL, TISSUE AND ORGAN CULTURE, vol.29, 1992 pages 125 - 133 P. COUTOS-THEVENOT ET AL. 'Somatic embryogenesis from grapevine cells. 1 - Improvement of embryo development by changes in culture conditions' cité dans la demande
- PLANT CELL, TISSUE AND ORGAN CULTURE, vol.9, 1987, DORDRECHT, NL pages 73 - 80 D.J. GRAY ET AL. 'Initiation and maintenance of long term somatic embryogenesis from anthers and ovaries of Vitis longii 'Microsperma'' cité dans la demande

## Description

La présente invention concerne un procédé destiné à favoriser la différenciation de cellules en culture, notamment au cours des processus de différenciation tissulaire, d'embryogénèse, ou d'organogénèse ; elle concerne plus particulièrement un procédé destiné à l'obtention d'embryons à partir de culture de cellules somatiques végétales en utilisant une enzyme protéolytique et l'application de ce procédé à la regénération de plantes, en particulier de la vigne.

Le principe de l'obtention d'embryons à partir de culture de cellules somatiques in vitro est bien connu, en théorie. On peut se reporter en particulier à la publication de D.J. Gray & J.A. Mortensen dans la revue Plant Cell, Tissues and Organ Culture (1987) vol. 9 p. 73-80. En ce qui concerne en particulier les embryons somatiques de vigne, on pourra se reporter au document US 4 532 733. Si l'embryogénèse somatique est praticable pour certaines espèces de plantes, pour d'autres par contre ce procédé est très difficile, voire impossible à mettre en oeuvre.

Pour comprendre les problèmes posés il faut rappeler brièvement les principes de culture de cellules somatiques destinées à la formation d'embryons.

L'embryogénèse somatique comporte essentiellement deux étapes ;
1- l'induction du potentiel embryogène par des auxines exogènes, généralement en fortes concentrations, qui permettent l'apparition d'agrégats (ou masses) proembryogènes (PEM) qui correspondent à des groupes de 10 à 50 cellules, notamment méristématiques.
2- le transfert de ces cellules sur des milieux exempts d'auxines qui conduisent à la formation d'embryons somatiques à partir de ces PEM, en suivant les différents stades d'évolution de l'embryogénèse végétale. Ces stades d'évolution sont généralement caractérisés par une forme particulière. Ainsi dans le cas de la vigne il est habituel de distinguer successivement les stades suivants : globules, coeur, torpille et plantule.

Pour certaines espèces végétales, l'obtention d'embryons somatiques s'avère très difficile, voire impossible. Par exemple, avec certains cultivars de vigne, les capacités embryogènes des cellules somatiques peuvent être stoppées, la plupart du temps au stade de la formation des agrégats proembryogènes, ou bien, le développement des embryons est bloqué, notamment au stade globulaire.

Ceci est d'autant plus gênant qu'il existe, en particulier pour la vigne, un très grand besoin d'obtention à grande échelle d'embryons somatiques, in vitro. En effet, les méthodes in vivo de production d'embryons sont souvent insuffisantes et non souhaitables, car elles conduisent en règle générale à des recombinaisons génétiques, ce qui entraîne la perte des caractères génétiques originaux du cépage ou du porte-greffe que l'on souhaite multiplier.

La demande EP 281375, a proposé l'addition de calmoduline et de calcium à une culture cellulaire pour favoriser la différenciation des racines et des embryons.

La demande EP 455597 décrit une méthode de stimulation de la croissance de l'embryogénèse des plantes cultivées in vitro, par addition d'arabino-galactane protéines. Il a cependant été démontré (de Vries et al, 1989, Proceedings of the International Symposium of Biotechnology for Major Crops (A.D.E.B.I.O. ed.), pages 22-23) qu'une glycoprotéine extracellulaire de 52/54 kDa induit un arrêt du développement embryonnaire au stade coeur.

La présente invention repose sur la mise en évidence de l'action d'enzymes protéolytiques permettant d'assurer ou de stimuler la différenciation cellulaire, et notamment l'embryogènèse somatique, notamment à partir de cellules somatiques de vigne.

En effet, de manière surprenante, la Demanderesse a mis en évidence que le développement des embryons dans des cultures de cellules somatiques peut être accéléré par l'addition d'une enzyme protéolytique. L'inhibition du développement des embryons habituellement observée quand la densité cellulaire augmente est levée par l'introduction d'au moins une enzyme protéolytique, en particulier choisie dans le groupe des protéases de type sérine comme la trypsine et la chymotrypsine, particulièrement pour les cultures dans laquelle la densité cellulaire initiale est faible ou modérée. Par exemple, pour 1 µg d'enzyme protéolytique par millilitre de milieu, l'efficacité est meilleure pour une densité inférieure ou égale à 0,4 de PCV (Packed Cell Volume = unité de volume cellulaire sédimenté à 1 x g) par millilitre de milieu.

C'est pourquoi la présente invention a pour objet un procédé pour favoriser la différenciation de cellules végétales en culture, notamment en vue de la différenciation tissulaire, de l'embryogénèse, ou de l'organogénèse, caractérisé en ce qu'on introduit dans le milieu de culture au moins une enzyme protéolytique en concentration efficace pour obtenir la différenciation des cellules.

Suivant un mode particulier de réalistion du procédé, la présente invention concerne un procédé d'obtention d'embryons somatiques végétaux à partir d'une culture de cellules somatiques, caractérisée en ce qu'on ajoute au milieu de culture au moins une enzyme protéolytique, en concentration efficace pour assurer l'embryogénèse.

Dans ce qui suit, on utilisera de façon générale le terme maturation pour désigner les phénomènes de développement et de germination de l'embryon végétal somatique, passant ainsi par les stades de développement suivants : globule, coeur,torpille et plantule.

La technologie de culture cellulaire in vitro de cellules somatiques est connue et devra bien entendu être adaptée à chaque souche cellulaire en cause, les conditions physico-chimiques, milieux de culture et environnement étant adaptés de façon à permettre la multiplication et la formation d'agrégats proembryogènes, puis ensuite l'embryogénèse. Les milieux correspondants seront appelés "milieux de culture pour embryogénèse".

Le procédé selon la présente invention est particulièrement intéressant pour obtenir la maturation d'embryons surtout dans le cas où il s'agit de cellules transformées. De préférence, l'enzyme protéolytique est introduite dans le milieu exempt d'auxine à un stade précoce du développement cellulaire, c'est-à-dire avant que les embryons aient atteint le stade globulaire. Le procédé selon l'invention consiste alors à introduire l'enzyme protéolytique dans le milieu d'induction et/ou de développement des agrégats de cellules proembryogènes.

Ainsi, selon l'invention, l'enzyme protéolytique peut être introduite dans le milieu d'induction et/ou de développement des embryons, qui est, selon l'art antérieur, généralement exempt d'auxine, voire sensiblement exempt d'auxine.

Une efficacité optimale est obtenue quand le procédé de l'invention concerne des cultures réalisées, au moins partiellement, en l'absence de lumière.

Suivant un mode particulier de réalisation du procédé selon l'un ou l'autre des deux aspects précités, l'enzyme protéolytique est présente dans le milieu de culture à une concentration comprise entre 0,1 et 100 µg/ml de milieu, de préférence entre 1 et 10 µg/ml.

Ainsi, chez la vigne, dans de telles conditions avec l'addition de 1 µg/ml de trypsine dans le milieu de développement et de maturation des embryons somatiques, on observe une amélioration de l'embryogénèse analogue à celle obtenue par la technique de sous-cultures journalières de Coutos-Thevenot et al(Plant Cell Tiss. Org. Cult. 29, 1992) et un nombre de plantules comparables.

Avantageusement, la densité initiale d'agrégats de cellules dans le milieu de culture est comprise entre 0,1 et 5 µg de PCV par ml de milieu de culture, de préférence cette densité est comprise entre 0,1 et 0,5 µg de PCV par ml de milieu de culture.

Suivant un autre mode particulier selon l'un ou l'autre de ces deux aspects, la culture de cellules précitée est une culture de cellules de vigne. Dans ce cas, le procédé selon l'invention convient particulièrement.

Avantageusement, l'enzyme protéolytique est choisie dans le groupe comprenant des protéases de type serine comme la trypsine ou la chymotrypsine.

De préférence encore, la protéase est la trypsine.

Une mise en oeuvre particulièrement avantageuse du procédé de l'invention consiste à introduire l'enzyme protéolitique dans des milieux de culture de cellules transformées, notamment de cellules de vigne, surtout dans le cas où la souche cellulaire présente une embryogénèse somatique perturbée.

Plusieurs techniques de transformation génétique sont connues, par exemple, la transformation par biolistique (par canon à particules) ou par des vecteurs de clonage appropriés, tels que des vecteurs dérivés d'Agrobactérium ou par d'autres systèmes de type viraux. Dans ce cas, l'étape de multiplication qui suit habituellement celle de la transformation, n'a pas forcément lieu d'être, en particulier en raison du fait que des cellules transformées pourront être directement développées en embryons, la sélection des embryons transformés se faisant alors au cours de ce développement, par des techniques bien connues, telle que la résistance à un antibiotique déterminé.

Suivant un mode avantageux de mise en oeuvre de l'invention, le milieu de culture de cellules précité, en particulier le milieu d'induction et/ou de développement des embryons, dans lequel est introduite l'enzyme protéolytique précitée, est également additionné d'un agent de stimulation de la différenciation cellulaire, en particulier de l'embryogénèse, telle qu'une protéine, notamment de type protéine de transfert de lipides (LTP), provenant par exemple d'une culture d'embryons somatiques de vigne telle que décrite dans la demande de brevet français n° 92-15044

La Demanderesse a en effet constaté que de telles protéines n'étaient généralement pas dégradées par les enzymes protéolytiques utilisées dans le cadre de la présente invention.

Selon une variante préférée du procédé de l'invention, applicable avantageusement au processus d'embryogénèse de la vigne, on utilise de la trypsine a titre d'enzyme protéolytique, que l'on additionne à un milieu de culture contenant une LTP de masse molaire de 9 kDa environ, telle que décrite dans la demande de brevet français n°92-15044.

Les exemples ci-après sont destinés à illustrer d'autres caractéristiques et avantages de la présente invention, sans toutefois en limiter la portée.

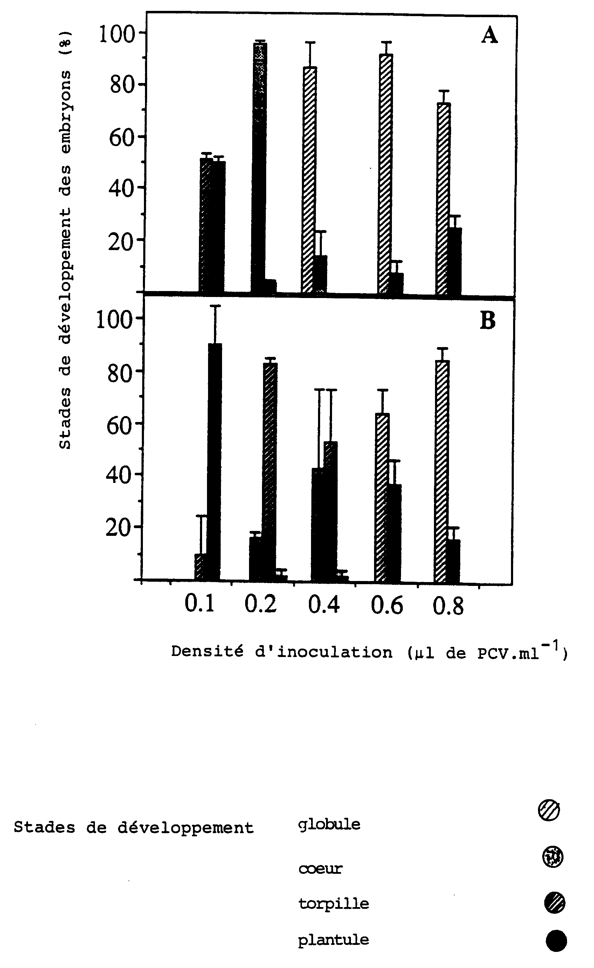

Dans ces exemples, on se référera à la figure en annexe, qui représente l'influence de l'addition de trypsine exogène sur le développement d'embryons dans des cultures à différentes densités d'inoculation.

### Exemple 1

### Influence de la concentration en trypsine sur le développement d'embryons de vignes

### Origine de la culture

On utilise comme source d'agrégats de cellules embryogènes, la culture de cellules embryogènes de porte-greffe de vigne 41 B déposée à l'European Collection of Animal Cell Cultures sous le n° PL 92042917 décrite dans la demande internationale PCT déposée le 11 mai 1993 sous le numéro PCT/FR93/00456. Pour initier les cultures d'embryons dans un milieu dépourvu d'auxine, la suspension cellulaire est filtrée successivement sur des tamis de 500 µm et 200 µm, et la fraction retenue sur 200 µm est lavée trois fois dans un milieu de culture dépourvu d'auxine, selon le protocole décrit précédemment. Les densités de population cellulaire au moment de l'inoculation déterminée par le PCV (Packed Cell Volume, unité de volume cellulaire sédimenté à 1 x g) sont comprises entre 0,05 et 1 µl de PCV.mL⁻¹ . La correspondance PCV-densité cellulaire est assurée par des numérations faites par la méthode de dispersion dans l'acide chromique.

### Modulation de l'embryogénèse par des complémentations en trypsine

La complémentation au début de la culture en milieu exempt d'auxine est effectuée avec la trypsine, dans des fioles d'Erlenmeyer de 50 mL, les stades embryonnaires sont rapportés après 15 jours de culture réalisée sans aucune sous-culture journalière, à la différence de la culture témoin décrite ci-après. La trypsine, disponible commercialement auprès de la société américaine Sigma, Saint-Louis, Missouri, est ajoutée à des concentrations de 0,05 à 10 µg.mL⁻¹. Un témoin positif de développement normal de l'embryon est constitué par des sous-cultures journalières comme décrit par Coutos-Thévenot et al (Plant Cell Tiss. Org. Cult., (1992) 29 125-133), avec une légère modification : les densités de population cellulaire ne sont pas réajustées au cours de l'expérience. Les cultures sont effectuées à 26° C à l'obscurité sous agitation orbitale (120 rpm).

Après 15 jours de culture, on compte le nombre d'embryons qui ont germé en plantules.

Les résultats sont présentés dans le tableau 1. On observe ainsi, que la trypsine provoque une accélération du développement des embryons, exprimés en nombre de plantules développées après 15 jours de culture inoculés initialement à la densité de 0,1 µg de PCV par ml de milieu.

**Tableau**

| **Effet de la trypsine exogène sur l'embryogénèse somatique.** | |
|---|---|
| Trypsine (µg/mL-¹) | Plantule (moyenne ± SE) |
| sc | 36,7±11,8 |
| 0 | 16,5±15,6 |
| 1 | 30,0±15,3 |
| 2 | 71,0±40,7 |
| 10 | 152,7±63,9 |
| sc : contrôle constitué par des sous-cultures journalières des l'embryons en présence de milieu frais. | |

Ainsi, l'ajout de trypsine constitue un moyen particulièrement précieux pour favoriser et accélérer le développement d'embryons somatiques végétaux, notamment dans le cadre d'un processus de régénération de plantes, en particulier de la vigne.

### Exemple 2

### Influence de la densité d'inoculation d'une culture d'embryons de vignes sur l'effet stimulant de la trypsine

On opère selon les conditions de l'exemple 1 avec une concentration de trypsine de 4 µg/ml et une densité d'inoculation initiale variable respectivement : 0,1 ; 0,2 ; 0,4 ; 0,6 ; 0,8 µl de PCV par ml de milieu. Six expériences indépendantes ont été pratiquées. Pour chaque expérience, quatre répétitions ont été réalisées.

Les effets sur le développement des embryons sont évalués après 15 jours de culture, et exprimés en pourcentage pour chaque stade de développement par rapport au nombre total d'embryons dénombrés, d'une part pour des cultures de contrôle (A), et d'autre part, pour des cultures (B) additionnées de trypsine. Les résultats sont rassemblés sur la figure annexée. Sur cette figure, la déviation standard est représentée par une petite barre.

On observe une stimulation du développement des embryons, qui est particulièrement sensible aux densités les plus faibles 0,1; 0,2 ; 0,4 par rapport à la culture témoin. En effet, on observe une quantité de plantules et d'embryons au stade torpille beaucoup plus importante à ces densités, ce qui traduit clairement une accélération dans le développement de l'embryogénèse.

## Revendications

1. Procédé pour favoriser la différenciation de cellules végétales en culture, notamment en vue de la différenciation tissulaire, de l'embryogénèse, et/ou de l'organogénèse, caractérisé en ce qu'on introduit dans le milieu de culture au moins une enzyme protéolytique en concentration efficace pour obtenir la différenciation des cellules.

2. Procédé pour favoriser la différenciation de cellules végétales en culture en vue de l'embryogénèse selon la revendication 1, caractérisé en ce qu'il s'agit d'un procédé d'obtention d'embryons somatiques végétaux à partir d'une culture de cellules somatiques, dans lequel on ajoute au milieu de culture, au moins une enzyme protéolytique en concentration efficace pour assurer l'embryogénèse.

3. Procédé d'obtention d'embryons somatiques végétaux selon la revendication 2, caractérisé en ce que l'enzyme protéolytique est ajoutée dans le milieu d'induction et/ou de développement des embryons.

4. Procédé d'obtention d'embryons somatiques végétaux selon la revendication 3, caractérisé en ce que le milieu d'induction et/ou de développement des embryons est sensiblement exempt d'auxine.

5. Procédé selon l'une des revendications 1 à 4, caractérisé en ce que la protéase est présente dans le milieu de culture à une concentration comprise entre 0,1 et 100 µg/ml de milieu, de préférence entre 1 et 10 µg/ml.

6. Procédé selon l'une des revendications 1 à 5, caractérisé en ce que l'enzyme protéolytique est choisie dans le groupe comprenant des protéases de type serine comme la trypsine ou la chymotrypsine.

7. Procédé selon la revendication 6, caractérisé en ce que la protéase est la trypsine.

8. Procédé selon l'une des revendications 1 à 7, caractérisé en ce que la densité d'inoculation de la culture en agrégat de cellules est comprise entre 0,1 et 5 µg de PCV (volume cellulaire sédimenté) par millilitre de milieu de culture et de préférence entre 0,1 et 0,5 µg de PCV/ml de milieu de culture.

9. Procédé selon l'une des revendications 1 à 8, caractérisé en ce que la culture est réalisée au moins partiellement, en l'absence de lumière.

10. Procédé selon l'une des revendications 1 à 9, caractérisé en ce que les cellules sont des cellules génétiquement transformées.

11. Procédé selon l'une des revendications 1 à 10, caractérisé en ce que la culture cellulaire est une culture de cellules de vigne.

12. Procédé selon l'une des revendications 1 à 11, caractérisé en ce que le milieu de culture de cellules, en particulier le milieu d'induction et/ou de développement des embryons, dans lequel est introduite l'enzyme protéolytique précitée est également additionné d'un agent de stimulation de la différenciation cellulaire, en particulier de l'embryogénèse, telle qu'une protéine, notamment du type protéine de transfert de lipide (LTP).

13. Procédé selon la revendication 12, caractérisé en ce que l'enzyme protéolytique est la trypsine et en ce que l'agent de stimulation est une LTP de masse molaire d'environ 9 kDa, provenant par exemple d'une culture d'embryons somatiques de vigne.

## Claims

1. Method for favouring the differentiation of plant cells in culture, in particular with a view to tissue differentiation, embryogenesis and/or organogenesis, characterized in that at least one proteolytic enzyme is introduced into the culture medium at a concentration which is effective for obtaining differentiation of the cells.

2. Method for favouring the differentiation of plant cells in culture with a view to embryogenesis according to Claim 1, characterized in that it is a method for obtaining plant somatic embryos from a culture of somatic cells, in which at least one proteolytic enzyme is added to the culture medium at a concentration which is effective for ensuring embryogenesis.

3. Method for obtaining plant somatic embryos according to Claim 2, characterized in that the proteolytic enzyme is added to the medium for inducing and/or developing the embryos.

4. Method for obtaining plant somatic embryos according to Claim 3, characterized in that the medium for inducing and/or developing the embryos is essentially free of auxin.

5. Method according to one of Claims 1 to 4, characterized in that the protease is present in the culture medium at a concentration of between 0.1 and 100 µg/ml of medium, preferably between 1 and 10 µg/ml.

6. Method according to one of Claims 1 to 5, characterized in that the proteolytic enzyme is selected from the group comprising proteases of the serine type, such as trypsin or chymotrypsin.

7. Method according to Claim 6, characterized in that the protease is trypsin.

8. Method according to one of Claims 1 to 7, characterized in that the density at which the culture is inoculated with cell aggregate is between 0.1 and 5 µl of PCV (sedimented cell volume) per millilitre of culture medium, and preferably between 0.1 and 0.5 µl of PCV/ml of culture medium.

9. Method according to one of Claims 1 to 8, characterized in that the culture is carried out, at least partially, in the absence of light.

10. Method according to one of Claims 1 to 9, characterized in that the cells are genetically transformed cells.

11. Method according to one of Claims 1 to 10, characterized in that the cell culture is a culture of grapevine cells.

12. Method according to one of Claims 1 to 11, characterized in that the cell culture medium, in particular the medium for inducing and/or developing the embryos, into which medium the abovementioned proteolytic enzyme is introduced, is also treated with an agent for stimulating cell differentiation, in particular embryogenesis, such as a protein, in particular of the lipid transfer protein (LTP) type.

13. Method according to Claim 12, characterized in that the proteolytic enzyme is trypsin and in that the stimulatory agent is an LTP of approximately 9 kDa molecular weight, which is derived, for example, from a culture of grapevine somatic embryos.

## Patentansprüche

1. Verfahren zur Förderung der Pflanzenzellendifferenzierung in Kultur, im besonderen in Hinblick auf die Gewebedifferenzierung, der Embryogenese und/oder der Organogenese, dadurch gekennzeichnet, daß man in das Kulturmedium Wenigstens ein proteolytisches Enzym in einer Konzentration einbringt, die zum Erhalt der Zelldifferenzierung wirksam ist.

2. Verfahren zur Förderung der Pflanzenzellendifferenzierung in Kultur in Hinblick auf die Embryogenese gemäß Anspruch 1, dadurch gekennzeichnet, daß es sich um Verfahren zum Erhalt von pflanzlichen somatischen Embryonen ausgehend von einer Kultur somatischer Zellen handelt, bei dem man dem Kulturmedium Wenigstens ein proteolytisches Enzym in einer zur Gewährleistung der Embryogenese wirksamen Konzentration zugibt.

3. Verfahren zum Erhalt von pflanzlichen somatischen Embryonen gemäß Anspruch 2, dadurch gekennzeichnet, daß das proteolytische Enzym in das Induktionsmedium und/oder das Entwicklungsmedium der Embryonen zugegeben wird.

4. Verfahren zum Erhalt von pflanzlichen somatischen Embryonen gemäß Anspruch 3, dadurch gekennzeichnet, daß das Induktionsmedium und/oder das Entwicklungsmedium der Embryonen frei von Auxin ist.

5. Verfahren nach einem der Ansprüche 1 bis 4, dadurch gekennzeichnet, daß die Protease in dem Kulturmedium in einer Konzentration zwischen 0,1 und 100 µg/ml Medium, bevorzugt zwischen 1 und 10 µg/ml, vorliegt.

6. Verfahren nach einem der Ansprüche 1 bis 5, dadurch gekennzeichnet, daß das proteolytische Enzym aus der Gruppe der Proteasen des Serintyps, wie Trypsin oder Chymotrypsin, ausgewahlt wird.

7. Verfahren gemäß Anspruch 6, dadurch gekennzeichnet, daß die Protease Trypsin ist.

8. Verfahren nach einem der Ansprüche 1 bis 7, dadurch gekennzeichnet, daß die Inokulationsdichte der Kultur im Zellaggregat zwischen 0,1 und 5µg PCV (sedimentiertes Zellvolumen) pro ml Kulturmedium, und bevorzugt zwischen 0,1 und 0,5 g PCV/ml Kulturmedium, beträgt.

9. Verfahren nach einem der Ansprüche 1 bis 8, dadurch gekennzeichnet, daß die Kultur wenigstens teilweise unter Lichtausschluß gehalten wird.

10. Verfahren nach einem der Ansprüche 1 bis 9, dadurch gekennzeichnet, daß es sich bei den Zellen um genetisch transformierte Zellen handelt.

11. Verfahren nach einem der Ansprüche 1 bis 10, dadurch gekennzeichnet, daß die Zellkultur eine Kultur von Weinzellen ist.

12. Verfahren nach einem der Ansprüche 1 bis 11, dadurch gekennzeichnet, daß das Zellkulturmedium, im besonderen das Induktionsmedium und/oder das Entwicklungsmedium der Embryonen, in welches das proteolytische Enzym eingebracht wird, außerdem mit einem Mittel zur Stimulation der Zelldifferenzierung, im besonderen der Embryogenese, wie einem Protein, insbesondere einem Lipidtransferprotein (LTP), versetzt ist.

13. Verfahren gemäß Anspruch 12, dadurch gekennzeichnet, daß das proteolytische Enzym Tripsin ist, und daß das Stimulationsmittel ein LTP mit einer molaren Masse von etwa 9 kDa, welches beispielsweise aus einer Kultur von somatischen Weinembryonen stammt, ist.
